# EUROPEAN PATENT APPLICATION

(11) **EP 1 038 955 A1**
(43) Date of publication of application: **27.09.2000**
(21) Application number: 00105543.3
(22) Date of filing: 16.03.2000
(51) Int. Cl.: C12N 15/10

(54) **Method for extraction and long-term storage of RNA**

(30) Priority: 17.03.1999 JP 7257199
(71) Applicant: SNOW BRAND MILK PRODUCTS, CO., LTD., Sapporo-shi Hokkaido 065-0043 (JP)
(72) Inventor: Imamura, Mio, Sapporo-shi, Hokkaido 060-0004 (JP); Itagaki, Yasuharu, Sapporo-shi 064-0811 (JP); Tanimoto, Morimasa, Kawagoe-shi, Saitama 350-1175 (JP)
(74) Representative: Forstmeyer, Dietmar, Dr. rer. nat., Dipl.-Chem.

(57) **Abstract**

A novel method for the extraction of RNA from milk and long-term storage of it is provided. The method comprises extracting milk with an RNA extracting agent containing phenol and guanidine thiocyanate to obtain RNA, and storing the RNA. The method is useful as a method that can quickly, simply, and efficiently extract RNA to be used in high sensitivity tests for bovine mastitis and store the extracted RNA for a long term.

## Description

### BACKGROUND OF THE INVENTION

### 1. Field of the Invention

The present invention relates to a novel method for the extraction of RNA from milk and for the long-term storage thereof. In particular, the present invention relates to a method for the extraction from milk of and long-term storage of RNA with an RNA extracting agent containing phenol and guanidine thiocyanate.

### 2. Description of the Related Art

Mastitis refers to inflammation of mamma or udder tissue caused by pathogenic microbes which invaded into the mamma or udder. When mammalians, particularly cows are affected with mastitis, a decrease in the amount of lactation occurs and in the worst cases shakeout of the affected cows is necessitated. Thus, economical loss caused by mastitis is very severe to dairying and milk manufacturing industry. Countermeasure for the mastitis as a rule is to find in earliest possible stage and to treat as early as possible. The diagnosis for mastitis includes a diagnosis based on milk, more particularly a method for counting the number of somatic cells, PL test (Shalm, O.W., D.O.Noorlander (1957): Experiments and observations leading to developments of the California Mastitis Test. J. Amer. Vet. Med. Ass. 130. 199-204), etc. have been generally used. These are diagnostic methods utilizing the phenomena that when a cow is affected by mastitis, the number of somatic cells in the milk secreted by the cow increases or the pH of the milk is shifted to the side of alkalinity. However, the number of somatic cells in milk and the pH of milk are both factors that vary greatly depending on individual mammalians or on whether or not the mammalian lactates, so that it is considered insufficient as a method for finding mastitis in an early stage. Generally, mastitis milk appears to vary in the components of milk as compared with normal milk. Discriminative feature is that among others casein decreases as compared with normal milk. Utilizing this, attempts have been made to judge mastitis by the value of casein nitrogen amount/total nitrogen amount. However, this method suffered from a wide fluctuation of measured values, making accurate judgement difficult.

Variation in milk components is caused by various factors. One of them is considered to be a mechanism that causative pathogenic bacteria invade through mammary ducts into mamma or udder and adhere to epithelial cells of mammary gland to thereby inhibit the synthesis of milk components in the epithelial cells of mammary gland. That is, it is considered that measurement of variation in the ability of the epithelial cells of mammary gland to synthesize milk components, for example, expression amount of casein protein in whatsoever method will make it possible to find mastitis in an early stage.

Recently, reverse transcription-polymerase chain reaction (RT-PCR) method utilizing the polymerase chain reaction (PCR method: U.S. Patent Nos. 4,683,195 and 4,683,202) have been used as a method for detecting the transcription amount of mRNA with high sensitivity. This method has made it possible to determine proteins expressed in cells qualitatively or quantitatively with high sensitivity. However, it is practically impossible to cut out the mammary gland tissue from cows, which are industrial animals, for extracting RNA therefrom for the diagnosis of mastitis. Accordingly, there has been no report on extracting RNA from the epithelial cells of mammary gland without damaging the living body. For this reason, various attempts have been made to extract RNA from milk for diagnosis. To extract RNA from milk, it is necessary to inhibit the decomposition of RNA by ribonuclease (RNase) to a minimum level. Further, to extract RNA derived from the epithelial cells of mammary gland, milk must be centrifuged to obtain precipitate of the epithelial cells of mammary gland before RNA extraction is performed, since the number of epithelial cells of mammary gland in milk is very small as compared with other cells. In this case, the number of epithelial cells of mammary gland in milk fluctuates greatly from sample to sample so that it is necessary to measure the amount of mRNA in the protein that is expressed only in epithelial cells of mammary gland and use it as a standard for the comparison of the expression amount of casein. Cells in milk other than the epithelial cells of mammary gland include mainly blood-derived cells such as leukocytes but these do not express keratin protein. Keratin gene, which is a cell structural gene that specifically expresses only in epithelial-type cells, includes various types and the kind of keratin gene that expresses may vary depending on species and site. Therefore, the primer used as an index for the amount of epithelial cells of bovine mammary gland must be hybridized with keratin that is expressed in the epithelial cells of bovine mammary gland. Also, the amount of keratin to be expressed must correlate with the number of epithelial cells. Further, αₛ₁-casein is known to have four mutants so that in order to obtain a general-purpose detection method, the primer to be used must hybridize in any of the αₛ₁-casein mutants.

Under the circumstances, the inventors of the present invention have made intensive study with view to develop a method for preparing RNA from milk and a high accuracy detection method for mastitis utilizing RNA. As a result, they have found a method for preparing RNA derived from milk, which method involves cooling milk quickly to ice cold, diluting the milk with a buffer and centrifuging the diluted milk, repeating these procedures, if desired, and extracting RNA from the resulting cell pellet. Further, they have found a method for detecting mastitis simply, quickly, and at high accuracy utilizing milk, which method involves subjecting the RNA prepared by the above method to reverse-transcription-polymerase chain reaction (RT-PCR; Wang et al., Proc. Natl. Acad. Sci. USA, 86, pp. 9717-9721 (1989)) utilizing respective primers designed such that a portion of the keratin gene and a portion of αₛ₁-casein gene expressed are amplified in correlation with the number of epithelial cells of mammary gland.

When mastitis is examined using such a method, separation of DNA or RNA from milk is necessary. Conventionally, separation of nucleic acids from milk has been performed by centrifuging milk, discarding the supernatant, adding a nucleic acid extracting reagent to the residual somatic cell pellet to extract a desired nucleic acid. However, this method necessitates centrifugation treatment of raw milk and hence when milking is performed in a place where no centrifugal separation apparatus or the like appliance is available, transportation of milk to the place where such an appliance is present is necessary. Further, since a large amount of ribonuclease is present in milk, DNA or RNA in the somatic cells will undergo decomposition by the ribonuclease when the raw milk is kept as is for a long time, so that there is the possibility that the results will be affected thereby. Furthermore, freezing milk in order to inhibit the action of ribonuclease will destroy somatic cells by freezing thawing so that there is the fear that the decomposition of nucleic acids after the thawing. Accordingly, when raw milk was to be used for the tests, such milk must be used for the tests within 5 hours after the milking. The conventional method has the problems that it involves many operations such as dilution of milk or centrifugation for obtaining somatic cells and concomitantly there will occur damages to cells and in addition the operation is troublesome.

The somatic cells in milk include many blood cells such as leukocytes as well as epithelial cells of mammary gland. Means for simply and easily detecting gene expression for inflammation factor in such cells, if any, will be used in the diagnosis of disease such as mastitis by measuring its expression level. Further, in view of rapid development of somatic cell cloning techniques in recent years suggests an increase in necessity of making judgment on the level of gene for screening cows having favorable characters. In this occasion, if RNA is readily extracted from somatic cells in milk and the gene expression level for milk protein such as casein is detected, the judgment of screening on predetermined criteria can be performed quickly, in a simple and easy manner and efficiently.

### SUMMARY OF THE INVENTION

Under the circumstances, the inventors of the present invention have made intensive research on a method for extracting RNA from milk and a method for storing RNA for a long-term storage. As a result, they have found that by extracting RNA from milk using an RNA extracting agent containing phenol and guanidine thiocyanate, RNA can be extracted in a simple and easy manner efficiently and a long-term storage of the extracted RNA is possible. That is, an object of the present invention is to provide a novel method for extracting and storing RNA for use in the diagnosis of mastitis in a simple and easy manner efficiently.

The present invention relates to a method for the extraction of RNA from milk and long-term storage of it, comprising: extracting milk with an RNA extracting agent containing phenol and guanidine thiocyanate to obtain RNA; and storing the RNA.

This method provides a method for extracting RNA from milk in a simple and easy manner efficiently and storing the extracted RNA for a long period of time. The method of the present invention is useful as a method that can quickly, in a simple and easy manner, and efficiently extract RNA to be used particularly in high sensitivity tests for bovine mastitis and store the extracted RNA for a long term.

### DETAILED DESCRIPTION OF THE INVENTION

The present invention relates to a method for the extraction of RNA from milk and long-term storage of it, comprising extracting milk with an RNA extracting agent containing phenol and guanidine thiocyanate to obtain RNA and storing the RNA. The method for extracting RNA from milk and storing it for a long period of time only requires treating milk by addition of an RNA extracting agent containing phenol and guanidine thiocyanate to sample milk. On this occasion, The amount (by volume, hereafter the same) of RNA extracting agent containing phenol and guanidine thiocyanate to sample milk is preferably about 1:1 to about 1:5, particularly about 1:3. If the ratio is lower than the above range, RNA cannot be extracted and if it is higher than the above range, RNA undergoes denaturation. The RNA extracting agent containing phenol and guanidine thiocyanate is not particularly limited but is preferably ILOGEN-LS (NIPPON GENE Co.) or RNAsol (TEL TEST Co.). Treatment of milk with these agents enables extraction of RNA quickly, in a simple and easy manner and efficiently.

Further, rapid freezing of the resultant RNA sample to -80°C enables a long-term storage thereof for 3 days or longer. On this occasion, the method of freezing is not particularly limited. For example, use of cold ethanol obtained by adding dry ice to ethanol makes it possible that sample milk after milking is treated with a RNA extracting agent containing phenol and guanidine thiocyanate on the site to obtain RNA, which is immediately frozen in a simple and easy manner. As the RNA extracting agent, various agents are known in addition to the composition as used in the present invention. However, use of such does not enable extraction of RNA.

ISOGEN-LS is a reagent contemplated for extracting RNA from liquid samples and RNAzol is a reagent contemplated to extract RNA from samples such as tissues or cultured cells. The RNA extracting agent used in the present invention contains equilibrated acid phenol 40 to 80% and 3 to 5 M guanidine thiocyanate. Use of guanidine thiocyanate is to deactivate the RNA decomposing enzymes and at the same time solubilize the proteins in the cell and use of phenol is to separate RNA from proteins, lipids, and DNA.

RNA is prepared from the resultant samples by a conventional method performing centrifugation using an organic solvent. For example, a suitable amount of chloroform is added to the mixture of sample milk and the RNA extracting agent and ultracentrifugation is performed. On this occasion, the conditions of ultracentrifugation are not particularly limited so far as they are ultracentrifugation conditions used in ordinary RNA extraction. However, the conditions of 4°C and rotation of about 15,000 RPM are preferred. Next, the upper layer of the centrifuged sample is adopted, to which an equivalent amount of isopropyl alcohol is added. After mixing, the mixture is kept in a refrigerator to precipitate RNA. The sample was centrifuged (30 minutes) under the same conditions as above to obtain RNA precipitate of sample milk. To the precipitate was added a suitable amount of 70% ethanol and centrifuged again to increase the purity of RNA to obtain high purity RNA.

The RNA thus obtained is used for confirming the level of protein expression in milk cow. This can be used in the diagnosis of mastitis or screening upon creating clone animals.

### EXAMPLES

The present invention will be described specifically by examples. However, the present invention is not construed as being limited thereto.

### Example 1

### Extraction of RNA form milk

In a clean sampling vessel is milked 2 ml of milk from a lactating Holstein cow. Using a pipette, 250 µl aliquot was charged into a microtube to make sample milk. 750 µl of ISOGEN-LS (NIPPON GENE CO.) was added thereto and well mixed. In a state of mixed liquid, this was stored at 4°C, -20°C and -80°C, respectively, for at longest 3 days. Everyday, 100 µl of chloroform was added to each sample and the mixture was vigorously stirred, followed by holding it at room temperature for 10 minutes. And then centrifuged at 15,000 rpm for 15 minutes to obtain the upper layer, i.e., RNA layer was collected. To the obtained upper layer was added an equivalent amount of isopropyl alcohol and the microtube was reversed upside down, and held in a refrigerator for 30 minutes to precipitate RNA. Thereafter, the mixture was centrifuged at 15,000 rpm for 30 minutes to collect the precipitate of RNA, to which was added 1 ml of 70% ethanol. The resultant mixture was centrifuged at 15,000 rpm for 15 minutes to increase the purity of the RNA precipitate. The obtained RNA precipitate was air-dried lightly and was dissolved by addition of 50 µl of distilled water to make an RNA solution. The obtained RNA solution was measured for yield and purity using a spectrophotometer. Comparison was made with RNA extracted without storage as a control. Results are shown in Table 1.

Keratin and casein were subjected to RT-PCR and evaluation of samples was carried out. Results obtained are sown in Table 2.

**Table 1**

| Test Sample | RNA yield (µg) | 260/280¹⁾ |
|---|---|---|
| Control | 76.8 ± 31.8 | 1.60 ± 0.001 |
| | | |
| Stored at 4°C for 1 day | 7.6 ± 42.3 | 1.50 ± 0.004 |
| Stored at 4°C for 2 days | 17.3 ± 29.9 | 1.78 ± 0.008 |
| Stored at 4°C for 3 days | 8.8 ± 48.1 | 1.56 ± 0.008 |
| | | |
| Stored at -20°C for 1 day | 70.0 ± 16.7 | 1.63 ± 0.001 |
| Stored at -20°C for 2 days | 89.3 ± 46.3 | 1.68 ± 0.001 |
| Stored at -20°C for 3 days | 78.9 ± 32.5 | 1.62 ± 0.001 |
| | | |
| Stored at -80°C for 1 day | 61.8 ± 41.5 | 1.67 ± 0.004 |
| Stored at -80°C for 2 days | 79.6 ± 12.6 | 1.63 ± 0.000 |
| Stored at -80°C for 3 days | 94.1 ± 39.2 | 1.66 ± 0.014 |

| | | |
|---|---|---|
| 1) For assay of the purity of RNA, ratio of absorbances at 260 nm to 280 nm was obtained. | | |

**Table 2**

| Test Sample | Casein expression/keratin expression |
|---|---|
| Control | 1.32 ± 0.03 |
| | |
| Stored at 4°C for 1 day | -²⁾ |
| Stored at 4°C for 2 days | - |
| Stored at 4°C for 3 days | - |
| | |
| Stored at -20°C for 1 day | 1.41 ± 0.04 |
| Stored at -20°C for 2 days | 1.39 ± 0.02 |
| Stored at -20°C for 3 days | 1.45 ± 0.01 |
| | |
| Stored at -80°C for 1 day | 1.37 ± 0.05 |
| Stored at -80°C for 2 days | 1.27 ± 0.05 |
| Stored at -80°C for 3 days | 1.27 ± 0.13 |

| | |
|---|---|
| 2) No PCR reaction occurred nor band appeared. | |

From the results it can be seen that the yield of RNA fluctuated and the lot stored at 4°C showed a decrease in yield and a decrease in purity attributable to RNA decomposition were observed. However, the lot of freeze storage, substantially no fluctuation was observed, showing no significant difference between samples. RT-PCR did not take place in the lot stored at 4°C, which suggested that RNA be decomposed. However, RT-PCR could be performed for all the samples in the freeze-stored lot, with the ratio of casein expression/keratin expression which was substantially the same value as that of control under all the storage conditions. However, when stored at -20°C, changes in the result of RT-PCR occurred which would be attributable to partial decomposition of RNA, so that storage at -80°C is most appropriate for use as a sample. That is, it revealed that rapid freezing at 80°C enables a long-term storage for up to 3 days.

## Claims

1. A method for the extraction of RNA from milk and long-term storage of it, comprising: extracting milk with an RNA extracting agent containing phenol and guanidine thiocyanate to obtain RNA; and storing the RNA.

2. The method for the long-term storage of RNA as claimed in claim 1, wherein the extraction is performed at a volume ratio of milk and RNA extracting agent of 1:1 to 1:5.

3. The method for the long-term storage of RNA as claimed in claim 1 or 2, wherein storage is performed at about -80°C.
